# EUROPEAN PATENT SPECIFICATION

(11) **EP 1 961 728 B1**
(45) Date of publication and mention of the grant of the patent: **11.12.2013**
(21) Application number: 06834835.8
(22) Date of filing: 15.12.2006
(51) Int. Cl.: C07C 51/487, C07C 57/52, C07C 229/26, C07B 57/00

(54) **METHOD FOR PRODUCING OPTICALLY ACTIVE (4E)-5-CHLORO-2-ISOPROPYL-4-PENTENOIC ACID OR BASIC AMINO ACID SALT THEREOF**
VERFAHREN ZUR HERSTELLUNG OPTISCH AKTIVER (4E)-5-CHLOR-2-ISOPROPYL-4-PENTENSÄURE ODER EINES BASISCHEN AMINOSÄURESALZES DAVON
PROCEDE POUR PRODUIRE DE L'ACIDE (4E)-5-CHLORO-2-ISOPROPYL-4-PENTENOIQUE OPTIQUEMENT ACTIF OU UN SEL D'ACIDE AMINE BASIQUE DE CELUI-CI

(30) Priority: 16.12.2005 JP 2005362862; 12.06.2006 JP 2006162220
(43) Date of publication of application: 27.08.2008
(73) Proprietor: Asahi Glass Company, Limited, Chiyoda-ku Tokyo 100-8405 (JP)
(72) Inventor: MAKINO, Mayumi, Tokyo 100-8405 (JP); SAKATA, Kazuhisa, Tokyo 100-8405 (JP); MATSUMURA, Yasushi, Tokyo 100-8405 (JP)
(74) Representative: Müller-Boré & Partner Patentanwälte
(86) International application number: PCT/JP2006/325108
(87) International publication number: WO 2007/069745

(56) References cited:
- WO-A1-2006/099926
- JP-A- 2003 506 345

## Description

### TECHNICAL FIELD

The present invention relates to a method for producing an optically active (4E)-5-chloro-2-isopropyl-4-pentenoic acid, a basic amino acid salt thereof, or an optically active (4E)-5-chloro-2-isopropyl-4-pentenoic acid ester, which is useful as an intermediate for e.g. agrochemicals or medicines.

### BACKGROUND ART

For optical resolution of an optical isomer mixture such as a racemic modification, the following methods are, for example, known.
(1) Preferential crystallization of a racemic modification
(2) A diastereomeric method using a resolving agent
(3) A separation method by a column chromatography packed with an optically active material
(4) A separation method using stereospecificity of an enzymatic reaction
(5) A separation method using an optically active membrane

In these methods, there are no regularities established about a relation between the type of an optical isomer mixture and the suitable method for optical resolution of the optical isomer mixture. Therefore, when optical resolution of optical isomer mixtures is to be carried out, the method and the conditions need to be considered for every optical isomer mixture, and a pass-fail needs to be identified.

For optical resolution of an optical isomer mixture of (4E)-5-chloro-2-isopropyl-4-pentenoic acid, the following methods have, for example, been proposed.
(A) A separation method using stereospecificity of an emzymatic reaction using an esterase derived from a pig liver which recognizes R-isomer (Patent Document 1).
(B) A diastereomeric method using cinchonidine which is an optically active alkaloid, as a resolving agent,
wherein triethylamine is added to a racemic modification and cinchonidine, to precipitate a salt from tetrahydrofuran, followed by recrystallization twice in acetone (Patent Document 2).

However, each method had problems such that yield and optical purity were insufficient, and operation was complex.
Patent Document 1: WO 01/09079
Patent Document 2: WO 02/08172

### DISCLOSURE OF THE INVENTION

### OBJECT TO BE ACCOMPLISHED BY THE INVENTION

The present invention is to provide a method for producing an optically active (4E)-5-chloro-2-isopropyl-4-pentenoic acid, a basic amino acid salt thereof or an optically active (4E)-5-chloro-2-isopropyl-4-pentenoic acid ester with high yield and high optical purity by simple operation.

### MEANS TO ACCOMPLISH THE OBJECT

The present invention provides the following.
(1) A method for producing a basic amino acid salt of optically active (4E)-5-chloro-2-isopropyl-4-pentenoic acid, which comprises precipitating the basic amino acid of optically active (4E)-5-chloro-2-isopropyl-4-pentenoic acid from a solvent solution containing an optical isomer mixture of (4E)-5-chloro-2-isopropyl-4-pentenoic acid and an optically active basic amino acid or a salt thereof.
(2) The method according to the above (1), wherein the solvent solution is a solvent solution formed by dissolving the optical isomer mixture of (4E)-5-chloro-2-isopropyl-4-pentenoic acid and the optically active basic amino acid or a salt thereof, in a solvent, or a solvent solution formed by dissolving, in the solvent, a product formed by reacting the optically active basic amino acid or a salt thereof with the optical isomer mixture of (4E)-5-chloro-2-isopropyl-4-pentenoic acid.
(3) The method according to the above (1) or (2), wherein the optical isomer mixture of (4E)-5-chloro-2-isopropyl-4-pentenoic acid is a racemic modification of (4E)-5-chloro-2-isopropyl-4-pentenoic acid.
(4) The method according to any one of the above (1) to (3), wherein the optically active basic amino acid is L-(+)-lysine.
(5) The method according to any one of the above (1) to (4), wherein the optically active (4E)-5-chloro-2-isopropyl-4-pentenoic acid is S-isomer.
(6) The method according to any one of the above (1) to (5), wherein the solvent solution is a solution of solvent containing an alcohol.
(7) The method according to any one of the above (1) to (5), wherein the solvent solution is a solution of solvent containing a ketone.
(8) The method according to any one of the above (1) to (5), wherein the solvent solution is a solution of solvent containing an alcohol and a ketone.
(9) The method according to any one of the above (1) to (8) further comprising the step of dissolving the basic amino acid salt of optically active (4E)-5-chloro-2-isopropyl-4-pentenoic acid in a solvent to obtain a solvent solution of the basic amino acid salt of optically active (4E)-5-chloro-2-isopropyl-4-pentenoic acid, and then, precipitating from the solvent solution, the basic amino acid salt of optically active (4E)-5-chloro-2-isopropyl-4-pentenoic acid.
(10) The method according to any one of the above (1) to (9) further comprising subjecting the basic amino acid of optically active (4E)-5-chloro-2-isopropyl-4-pentenoic acid to a desalting reaction to yield an optically active (4E)-5-chloro-2-isopropyl-4-pentenoic acid.
(11) The method according to the above (10) further comprising reacting an optically active basic amino acid with the optically active (4E)-5-chloro-2-isopropyl-4-pentenoic acid.
(12) A method for producing an optically active (4E)-5-chloro-2-isopropyl-4-pentenoic acid, which comprises subjecting a salt of optically active (4E)-5-chloro-2-isopropyl-4-pentenoic acid with L-(+)-lysine, to a desalting reaction.
(13) A method for producing (S)-(4E)-5-chloro-2-isopropyl-4-pentenoic acid, which comprises subjecting a salt of (S)-(4E)-5-chloro-2-isopropyl-4-pentenoic acid with L-(+)-lysine, to a desalting reaction.
(14) The method according to any one of the above 10 or 12 further comprising reacting the optically active (4E)-5-chloro-2-isopropyl-4-pentenoic acid with an alcohol to yield an optically active (4E)-5-chloro-2-isopropyl-4-pentenoic acid ester.
(15) The method according to the above (14), wherein the optically active (4E)-5-chloro-2-isopropyl-4-pentenoic acid is S-isomer.
(16) A salt of optically active (4E)-5-chloro-2-isopropyl-4-pentenoic acid with a basic amino acid.
(17) The salt according to the above (16), wherein the salt is a salt of (S)-(4E)-5-chloro-2-isopropyl-4-pentenoic acid with L-(+)-lysine.

### EFFECTS OF THE INVENTION

By the present invention, it is possible to produce an optically active (4E)-5-chloro-2-isopropyl-4-pentenoic acid, a basic amino acid salt thereof or an optically active (4E)-5-chloro-2-isopropyl-4-pentenoic acid ester with high yield and high optical purity by simple operation.

### BEST MODE FOR CARRYING OUT THE INVENTION

In the present invention, production of (4E)-5-chloro-2-isopropyl-4-pentenoic acid (hereinafter referred to as 4-pentenoic acid derivative), a basic amino acid salt thereof (hereinafter referred to as 4-pentenoic acid derivative salt) and (4E)-5-chloro-2-isopropyl-4-pentenoic acid ester (hereinafter referred to as 4-pentenoic acid ester derivative), may be carried out by conducting the following respective steps (a) to (f) sequentially.
(a) Preparing a solvent solution containing an optical isomer mixture of 4-pentenoic acid derivative and an optically active basic amino acid or a salt thereof.
(b) Precipitating an optically active 4-pentenoic acid derivative salt from the solvent solution.
(c) As the case requires, reprecipitating the optically active 4-pentenoic acid derivative salt obtained in the above step (b).
(d) As the case requires, an optically active 4-pentenoic acid derivative is obtained by desalting the 4-pentenoic acid derivative salt obtained in the above step (b) or (c).
(e) As the case requires, purifying the optically active 4-pentenoic acid derivative obtained in the above step (d).
(f) Obtaining an optically active 4-pentenoic acid ester derivative by carrying out an esterification reaction of the optically active 4-pentenoic acid derivative obtained in the above step (d) or (e) with an alcohol.

In the above step (a), it is possible to prepare the solvent solution, for example, by a method (a-1) or (a-2).
(a-1) A solvent solution is formed by dissolving, in a solvent, an optical isomer mixture of 4-pentenoic acid derivative, and an optically active basic amino acid or a salt thereof.
(a-2) A product formed by reacting an optical isomer mixture of 4-pentenoic acid derivative with an optically active basic amino acid or a salt thereof, is dissolved in a solvent.

The optical isomer mixture of 4-pentenoic acid derivative in the step (a), includes a mixture of R-isomer and S-isomer of 4-pentenoic acid derivative (especially, a racemic modification which is a 1:1 (molar ratio) mixture of R-isomer to S-isomer is preferred). Further, the optical isomer mixture may further contain one or more optical isomers of (4Z)-5-chloro-2-isopropyl-4-pentenoic acid, which are 4-position isomers.

The racemic modification of 4-pentenoic acid derivative may, for example, be formed by the method described in WO 2004/052828.

The optically active basic amino acid may, for example, be optically active lysine, optically active arginine or optically active histidine. From the view point of availability and cost, L-(+)-lysine is preferred.

The L-(+)-lysine or its salt may be obtained in the form of e.g. an anhydride, a monohydrate, a hydrate, a 50% aqueous solution, a monohydrochloride or a dihydrochloride.

The amount of the optically active basic amino acid or its salt is preferably from 0.1 to 5 times by mol, more preferably from 0.8 to 3 times by mol, further preferably from 0.9 to 2 times by mol, based on the amount of 4-pentenoic acid derivative having the desired steric structure, which is contained in the optical isomer mixture. For example, when a racemic modification is to be optically resolved, 1/2 time by mol of the total amount of the racemic modification will be the amount of 4-pentenoic acid derivative having the desired steric structure.

As the solvent, water or an organic solvent is mentioned. The solvent may be a single solvent or a mixture of two or more solvents.

As the organic solvent, a solvent which does not react with the 4-pentenoic acid derivative, the optically active basic amino acid or the 4-pentenoic acid derivative salt, is preferred.

The solvent to be used is preferably the same one as used in after-mentioned precipitation and crystallization.

The organic solvent may, for example, be an aromatic hydrocarbon such as benzene, toluene or xylene; an ether such as diethyl ether, tert-butyl methyl ether, dioxane, tetrahydrofuran (THF) or dimethoxyethane; an alcohol such as methanol, ethanol, 2-propanol, 1-butanol, 2-butanol, 2-methyl propanol or 1,1-dimethyl ethanol; a glycol such as ethylene glycol or diethylene glycol; an ester such as ethyl acetate; a nitrile such as acetonitrile; an amide such as N,N-dimethylformamide; a sulfoxide such as dimethylsulfoxide; a halogenated hydrocarbon such as chloroform, dichloromethane, 1,2-dichloroethane or 1,1,2,2-tetrachloroethane; a ketone such as acetone or 2-butanone.

The organic solvent is preferably a single solvent of an alcohol, a single solvent of a ketone, or a mixture of an alcohol and a ketone, since the optically active 4-pentenoic acid derivative salt is thereby efficiently precipitated.

The alcohol is preferably a C₁₋₄ alcohol, particularly preferably methanol, ethanol, 1,1-dimethyl ethanol or 2-propanol.

As the ketone, acetone is particularly preferred.

When the alcohol is used as the organic solvent, one or more alcohols, a mixture thereof with water, or a mixture thereof with a ketone and water, is preferred. When methanol is used, it is preferred to use methanol alone, since the amount of the solvent may be lowered. Further, when ethanol, 1,1-dimethyl ethanol or 2-propanol is used, it is preferred to use it as a mixture with water or a mixture with acetone and water.

When a ketone is used as the organic solvent, a mixture of a ketone and water or a mixture of a ketone, an alcohol and water, is preferred. When acetone is used, it is preferred to use it as a mixture with water, and it is particularly preferred to use it as a mixture with water and 2-propanol.

When a mixture of an alcohol and water is used, a mixing ratio varies depending on the type of the alcohol. Water is usually preferably more than 0 vol% and at most 20 vol%, based on the alcohol.

In a mixture of ethanol and water, water is preferably more than 0 vol% and at most 10 vol%, based on ethanol. In a mixture of 2-propanol and water, water is preferably from 2 vol% to 15 vol%, based on 2-propanol.

When a mixture of a ketone and water is used, a mixing ratio varies depending on the type of the ketone. Water is usually preferably more than 0 vol% and at most 20 vol%, based on the ketone. In a mixture of acetone and water, water is preferably from 2 vol% to 15 vol%, based on acetone.

When a mixture of a ketone, an alcohol and water is used, a mixing ratio varies depending on the types of the ketone and the alcohol. Water is usually preferably more than 0 vol% and at most 20 vol%, based on the ketone, and the alcohol is preferably more than 0 vol% and less than 100 vol%, based on the ketone. In a mixture of acetone, 2-propanol and water, water is preferably from 2 vol% to 15 vol%, based on acetone, and 2-propanol is preferably from 5 vol% to less than 100 vol%, based on acetone.

The amount of the organic solvent is usually preferably from 2 to 200 times by mass, more preferably from 4 to 50 times by mass, based on 4-pentenoic acid derivative having the desired steric structure.

The solvent solution may contain a base in order to carry out optical resolution.

The base may, for example, be an inorganic base such as sodium hydroxide, potassium hydroxide, potassium carbonate, sodium carbonate or sodium hydrogencarbonate; an organic base such as pyridine, triethylamine or N,N-dimethylaniline.

The amount of the base is preferably from 0.1 to 10 times by mol, more preferably from 0.2 to 4 times by mol, based on 4-pentenoic acid derivative having the desired steric structure, which is contained in an optical isomer mixture.

As the precipitation method of the above step (b), method (b-1) or (b-2) is mentioned.
(b-1) A method to utilize the difference in solubility, by the temperature, of the optically active 4-pentenoic acid derivative salt into a solvent.
(b-2) A method to evaporate a part of the solvent from the solvent solution.

As the step (b), method (b-1) is preferred. The precipitate may be crystal, non-crystal or a mixture thereof. Further, the step (b) in the present invention is preferably a step for precipitating a salt of (S)-4-pentenoic acid derivative with L-(+)-lysine.

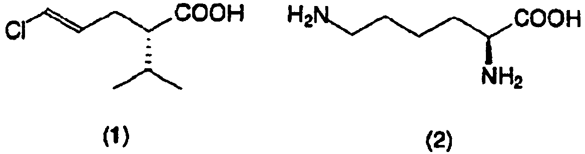

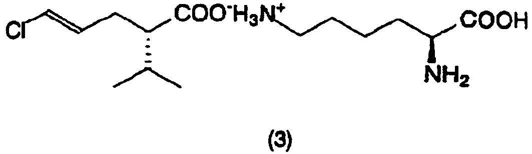

The above step (c) is a step to be optionally carried out, and it is preferably carried out when the optical purity of the precipitate of the optically active 4-pentenoic acid derivative salt, which is obtained in the above step (b), is lower than the desired purity. Particularly, when a racemic modification is used as the optical isomer mixture, the optical purity tends to be low, whereby it is preferred to carry out the step (c). Further, the step (c) in the present invention is preferably a step for reprecipitating a salt of (S)-4-pentenoic acid derivative with L-(+)-lysine.

In the step (c), the precipitate obtained in the step (b) is dissolved in a solvent to obtain a solvent solution of the 4-pentenoic acid derivative salt, and then, the optically active 4-pentenoic acid derivative salt is precipitated from the solvent solution. The precipitate formed in the step (c) may be crystal, non-crystal or a mixture thereof, and crystal is preferred.

The solvent may be similar to one used in the steps (a) and (b), and it is preferred to use an alcohol or a mixture of an alcohol and water. The preferred modes for the solvent are the same as the modes of the solvent described in the step (a). The solvent in the step (c) may be the same solvent as in the steps (a) and (b) or a different solvent, and it is preferred to be the same solvent.

The desalting reaction in the above step (d) is carried out by method (d-1) or (d-2).
(d-1) A method to contact the optically active 4-pentenoic acid derivative salt with a strong acid.
(d-2) A method to react the optically active 4-pentenoic acid derivative salt with a base.

The strong acid may, for example, be hydrochloric acid, sulfuric acid or nitric acid.

The base may, for example, be sodium hydroxide, potassium hydroxide, potassium carbonate or sodium hydrogencarbonate.

It is preferred to carry out the desalting reaction by method (d-1).

The method (d-1) may be carried out in an aqueous medium. The amount of water is preferably an amount wherein the resulting salt of the strong acid with the basic amino acid will dissolve sufficiently.

Further, the step (d) in the present invention is preferably a step for carrying out a desalting reaction of the salt of (S)-4-pentenoic acid derivative with L-(+)-lysine.

The majority of the optically active 4-pentenoic acid derivative obtained in the step (d) separates into an oily material, and a part is dissolved in an aqueous phase. The optically active 4-pentenoic acid derivative dissolved in the aqueous phase may be extracted with an organic solvent. The organic solvent may, for example, be a hydrocarbon such as hexane or cyclohexane; an aromatic hydrocarbon such as benzene, toluene or xylene; an ester such as ethyl acetate; an ether such as diethyl ether, tert-butyl methyl ether or tetrahydrofuran.

The above step (e) is a step to purify the optically active 4-pentenoic acid derivative obtained in the above step (d), as the case requires.

The step (e) is preferably carried out by the following methods of repeating a salt-forming reaction and desalting reaction. That is, the optically active 4-pentenoic acid derivative is reacted with the optically active basic amino acid to obtain an optically active 4-pentenoic acid derivative salt, and then, the optically active 4-petnanoic acid derivative salt, is subjected to the desalting reaction to obtain the optically active 4-pentenoic acid derivative.

The above step (f) is a step for reacting the optically active 4-pentenoic acid derivative obtained in the step (d) or (e), with an alcohol to obtain an optically active 4-pentenoic acid ester derivative.

The alcohol is preferably an alkanol having at most 6 carbon atoms, or an aralkanol having a total of at most 14 carbon atoms, more preferably a lower alkanol (alkanol having at most 4 carbon atoms), particularly preferably methanol or ethanol.

As the esterification reaction, a general dehydration-condensation method is widely used. For example, a method to carry out a reaction in the presence of an acid or a dehydrating agent, is mentioned. The esterification reaction is specifically preferably a method such that an alcohol is added to the optically active 4-pentenoic acid derivative, followed by stirring, and then, an acid is added.

The amount of the alcohol is preferably from 1 to 1×10² times by mol, particularly preferably from 1 to 50 times by mol, based on the optically active 4-pentenoic acid derivative. The acid may, for example, be sulfuric acid, hydrochloric acid or para-toluenesulfonic acid, and sulfuric acid is particularly preferred.

In the esterification reaction, it is preferred to adjust the amount of the acid and the reaction temperature to prevent racemization. The amount of the acid is preferably from 0.1 to 20 times by mol, based on the optically active 4-pentenoic acid derivative. The reaction temperature is preferably at a level of from 50 to 150°C, more preferably at a level of from 50 to 110°C.

The 4-pentenoic acid derivative contained in e.g. a waste liquid of the respective above steps (b), (c), (d) and (e), may be recovered and recycled. For example, (i) after the basic amino acid salt of S-isomer is precipitated in the step (b), R-isomer contained in the waste liquid is recovered and as the case requires, it is purified and used as an intermediate; (ii) a large amount of R-isomer is contained in the wasted liquid, and therefore, when S-isomer is required as an intermediate, the R-isomer is recovered and formed into a racemic modification, and it is reused as an optical isomer mixture for producing S-isomer.

The optically active basic amino acid may be recovered by adding a strong base (e.g. a sodium hydroxide solution) to the waste liquid in the step (d), followed by e.g. an extraction method.

The optically active 4-pentenoic acid derivative salt obtained in the method of the present invention, is a new compound, and it is useful as an intermediate for e.g. agrochemicals or medicines. As the 4-pentenoic acid derivative salt, a salt of (S)-4-pentenoic acid derivative with L-(+)-lysine is preferred, since it is more useful as an intermediate for e.g. agrochemicals or medicines.

The optical purity of the optically active 4-pentenoic acid derivative obtained in the method of the present invention is preferably at least 97%, more preferably at least 98%, further preferably at least 99%.

### EXAMPLE

Now, the present invention will be described in further detail with reference to Examples.

Hereinafter, "%" means "mass%" unless otherwise specified.

In Examples, gas chromatography is abbreviated as GC.

The structure of a compound was determined by comparing with known data.

The optical purity is measured by GC by using a column of Lipodex E 50 m × 0.25 mm (manufactured by Macherey-Nagel).

The NMR spectrum was measured by using a device with 300MHz, and tetramethyl silane was used as the internal standard. A chemical shift is shown by ppm, s represents singlet, t represents triplet, m represents multiplet, and the unit for a coupling constant (J) is Hz.

### EXAMPLE 1

Into a glass eggplant type flask, a suspension of L-(+)-lysine monohydrate (2.7 g, 16.5 mmol) in anhydrous ethanol (90 mL) was introduced. To the suspension, the racemic modification of 4-pentenoic acid derivative (5.3 g, 30 mmol) was added, and with stirring, it was heated for refluxing at 90°C for 1 hour to prepare a solvent solution. After completion of the heating, the solvent solution was left to cool to room temperature with stirring, whereby a white solid precipitated. The solvent solution was further stirred at around 7°C overnight.

The white solid was collected by filtration and washed with a mixture of ethanol and tert-butyl methyl ether (ethanol/tert-butyl methyl ether = 1:1 (volume ratio)), and it was dried under reduced pressure to obtain (S)-4-pentenoic acid derivative-(L)-lysine salt (3.75 g, 11.6 mmol). The yield from the racemic modification was 39%.

Into a glass eggplant type flask, (S)-4-pentenoic acid derivative-(L)-lysine salt (3.75 g) was introduced, and 2 mol/L hydrochloric acid (12 mL) was further added, followed by extraction with tert-butyl methyl ether (10 mL × twice). After washing the organic layer with a saturated sodium chloride aqueous solution (10 mL), it was concentrated under reduced pressure to obtain (S)-4-pentenoic acid derivative (2.00 g, 11.3 mmol). The yield of the 4-pentenoic acid derivative from the racemic modification was 38%.

To a solution having the (S)-4-pentenoic acid derivative (10 mg) dissolved in ethyl acetate (0.5 mL), methanol (0.3 mL) was added, and with stirring at room temperature, a hexane solution (0.15 mL) containing 0.60 mol/L of trimethylsilyl diazomethane was added. After stirring for 30 minutes at room temperature, the solvent was evaporated in a nitrogen stream to obtain a concentrated product. The concentrated product was diluted with ethyl acetate, and it was analyzed by GC analysis, whereby the optical purity was 98.3% for S-isomer and 1.7% for R-isomer.

### EXAMPLE 2

Into a glass vial bottle, the racemic modification of 4-pentenoic acid derivative (1.76 g, 10 mmol) was introduced. To the racemic modification, L-(+)-lysine anhydride (1.46 g, 10 mmol) and methanol (4 mL) were added, and the mixture was heated with stirring, on a hot plate until boiling to prepare a solvent solution. After completion of the heating, the solvent solution was left to cool to room temperature with stirring, whereby a white solid precipitated.

The white solid was collected by filtration and washed with a mixture of methanol and tert-butyl methyl ether (methanol/tert-butyl methyl ether = 1:1 (volume ratio)), to obtain (S)-4-pentenoic acid derivative-(L)-lysine salt.

Into a glass vial bottle, the (S)-4-pentenoic acid derivative-(L)-lysine salt (total amount) was introduced, and 2mol/L hydrochloric acid (4mL) was further added, followed by extraction with tert-butyl methyl ether (2 mL × 3 times). By concentrating the organic layer under reduced pressure, (S)-4-pentenoic acid derivative (469 mg, 2.66 mmol) was obtained. The yield of the 4-pentenoic acid derivative from the racemic modification was 27%.

The (S)-4-pentenoic acid derivative (10 mg) was subjected to the same preliminary treatment as in Example 1, and then it was analyzed by GC analysis, whereby the optical purity was 92.5% for S-isomer and 7.5% for R-isomer.

### EXAMPLE 3

Into a glass vial bottle, the (S)-4-pentenoic acid derivative (424 mg, 2.4 mmol) having an optical purity of 92.5%, obtained in Example 2, was introduced, and L-(+)-lysine anhydride (351 mg, 2.4 mmol) and methanol (3 mL) were further added, followed by heating with stirring, on a hot plate until boiling to prepare a solvent solution. The solvent solution was concentrated in a nitrogen stream, until methanol became 2 mL, and it was left to cool, whereby a white solid precipitated.

After cooling in a refrigerator overnight, the white solid was collected by filtration and washed with a mixture of methanol and tert-butyl methyl ether (methanol/tert-butyl methyl ether = 1:1 (volume ratio)), to obtain (S)-4-pentenoic acid derivative-(L)-lysine salt.

Into a glass vial bottle, the (S)-4-pentenoic acid derivative-(L)-lysine salt (total amount) was introduced, and 2mol/L hydrochloric acid (2 mL) was further added, followed by extraction with tert-butyl methyl ether (1 mL × twice). By concentrating the organic layer under reduced pressure, (S)-4-pentenoic acid derivative (303 mg, 1.7 mmol) was obtained. The yield from the (S)-4-pentenoic acid derivative having an optical purity of 92.5%, was 71%.

The obtained (S)-4-pentenoic acid derivative (10 mg) was subjected to the same preliminary treatment as in Example 1, and then it was analyzed by GC analysis, whereby the optical purity was 98.5% for S-isomer and 1.5% for R-isomer.

### EXAMPLE 4

Into a glass eggplant type flask, a suspension of L-(+)-lysine monohydrate (2.7 g, 16.5 mmol) in anhydrous ethanol (85 mL) was introduced. To the suspension, the racemic modification of 4-pentenoic acid derivative (5.3 g, 30 mmol) was added, and with stirring, it was heated for refluxing at 90°C for 1 hour to prepare a solvent solution. After completion of the heating, the solvent solution was left to cool to room temperature with stirring, whereby a white solid precipitated. The solvent solution was further stirred at around 7°C overnight.

The white solid was collected by filtration and washed with a mixture of ethanol and tert-butyl methyl ether (ethanol/tert-butyl methyl ether = 1:1 (volume ratio)), and it was dried under reduced pressure to obtain (S)-4-pentenoic acid derivative-(L)-lysine salt (3.86 g, 12.0 mmol). The yield from the racemic modification was 40%.

Into an Eppendorf tube, the (S)-4-pentenoic acid derivative-(L)-lysine salt (20 mg) was introduced, and 2 mol/L hydrochloric acid (0.5 mL) was further added, followed by extraction with ethyl acetate (0.5 mL). To the organic layer, 0.15 mL of methanol was added, and a hexane solution (0.15 mL) of 0.60 mol/L trimethyl diazomethane was added with stirring at room temperature. After stirring at room temperature for 1 hour, the solvent was evaporated in a nitrogen stream to obtain a concentrated product. The concentrated product was diluted with acetonitrile, and it was analyzed by GC analysis, whereby the optical purity was 96.7% for S-isomer and 3.3% for R-isomer.

### EXAMPLE 5

Into a glass eggplant type flask, the (S)-4-pentenoic acid derivative-(L)-lysine salt having an optical purity of 96.7%, obtained in Example 4 (3.86 g, 12.0 mmol), was introduced, and methanol (15 mL) and anhydrous ethanol (35 mL) were further added. With stirring, the mixture was heated for refluxing at 90°C for 1 hour to prepare a solvent solution. After completion of the heating, the solvent solution was left to cool to room temperature with stirring, whereby a white solid precipitated. The solvent solution was further stirred at around 7°C overnight.

The white solid was collected by filtration and washed with a mixture of ethanol and tert-butyl methyl ether (ethanol/tert-butyl methyl ether = 1:1 (volume ratio)), and it was dried under reduced pressure to obtain (S)-4-pentenoic acid derivative-(L)-lysine salt (3.08 g, 9.55 mmol). The yield from the (S)-4-pentenoic acid derivative-(L)-lysine salt having an optical purity of 96.7%, was 80%.

Into a glass eggplant type flask, the (S)-4-pentenoic acid derivative-(L)-lysine salt (3.06 g) was introduced, and 2 mol/L hydrochloric acid (10 mL) was further added, followed by extraction with tert-butyl methyl ether (10 mL × twice). After washing the organic layer with a saturated sodium chloride aqueous solution, it was concentrated under reduced pressure to obtain (S)-4-pentenoic acid derivative (1.65 g, 9.36 mmol). The yield from the (S)-4-pentenoic acid derivative-(L)-lysine salt having an optical purity of 96.7%, was 78%.

The obtained (S)-4-pentenoic acid derivative (10 mg) was subjected to the same preliminary treatment as in Example 1, and then it was analyzed by GC analysis, whereby the optical purity was 99.95% for S-isomer and 0.05% for R-isomer.

### EXAMPLE 6

Into a glass eggplant type flask, the racemic modification of 4-pentenoic acid derivative (5.3 g, 30 mmol) was introduced. To the racemic modification, L-(+)-lysine monohydrate (2.7 g, 16.5 mmol) and hydrous ethanol (95%, 25 mL) were further added, and with stirring, the mixture was heated for refluxing at 90°C for 1 hour to prepare a solvent solution. After completion of the heating, the solvent solution was left to cool to room temperature with stirring, whereby a white solid precipitated. The solvent solution was further stirred at around 7°C overnight.

The white solid was collected by filtration and washed with a mixture of ethanol and tert-butyl methyl ether (ethanol/tert-butyl methyl ether = 1:1 (volume ratio)), and it was dried under reduced pressure to obtain (S)-4-pentenoic acid derivative-(L)-lysine salt (2.33 g, 7.2 mmol). The yield from the racemic modification was 24%.

The melting point: 173.5 to 174.5°C.

¹H-NMR (CD₃OD) (δ value) : 0.77 (6H, t, J=6.9 Hz), 1.23 to 1.41 (2H, m), 1.50 to 1.64 (3H, m), 1.73 to 1.86 (3H, m), 1.94 to 2.05 (1H, m), 2.15 to 2.24 (1H, m), 2.89 (2H, t, J=7.2 Hz), 3.62 (1H, t, J=6.0 Hz), 5.74 to 5.95 (2H, m).

The obtained (S)-4-pentenoic acid derivative-(L)-lysine salt (20 mg) was subjected to the same preliminary treatment as in Example 4, and then it was analyzed by GC analysis, whereby the optical purity was 99.1% for S-isomer and 0.9% for R-isomer.

### EXAMPLE 7

Into a glass eggplant type flask, a 50% aqueous solution of L-(+)-lysine (4.8 g, 16.5 mmol) was introduced, and the racemic modification of 4-pentenoic acid derivative (5.3 g, 30 mmol) and 2-propanol (50 mL) were added. With stirring, the mixture was heated for refluxing at 90°C for 15 minutes to prepare a solvent solution. After completion of the heating, the solvent solution was left to cool to room temperature with stirring, whereby a white solid precipitated. The solvent solution was further stirred at around 7°C overnight.

The white solid was collected by filtration and washed with 2-propanol, and it was dried under reduced pressure to obtain (S)-4-pentenoic acid derivative-(L)-lysine salt (3.85 g, 11.9 mmol). The yield from the racemic modification was 40%.

The obtained (S)-4-pentenoic acid derivative-(L)-lysine salt was subjected to the same preliminary treatment as in Example 4, and then it was analyzed by GC analysis, whereby the optical purity was 99.1% for S-isomer and 0.9% for R-isomer.

### EXAMPLE 8

Into a four-necked round bottom flask, a 50.7% aqueous solution of L-(+)-lysine (26.9 g, 93.4 mmol) was introduced, and the racemic modification of 4-pentenoic acid derivative (30.0 g, 170 mmol) and 2-propanol (226 mL) were added. With stirring, the mixture was heated for refluxing at 90°C for 15 minutes to prepare a solvent solution. After completion of the heating, the solvent solution was left to cool to room temperature with stirring, whereby a white solid precipitated. The solvent solution was further stirred at around -10°C overnight.

The white solid was collected by filtration and washed with 7% hydrous 2-propanol, and it was dried under reduced pressure to obtain (S)-4-pentenoic acid derivative-(L)-lysine salt (20.1 g, 62.2 mmol). The yield from the racemic modification was 37%.

The obtained (S)-4-pentenoic acid derivative-(L)-lysine salt (20 mg) was subjected to the same preliminary treatment as in Example 4, and then it was analyzed by GC analysis, whereby the optical purity was 99.1% for S-isomer and 0.9% for R-isomer.

### EXAMPLE 9

Into a four-necked round bottom flask, the (S)-4-pentenoic acid derivative-(L)-lysine salt (274.1 g, 849 mmol) was introduced, and 35% hydrochloric acid (114 mL) was further added, followed by extraction with tert-butyl methyl ether (741 mL × twice). By concentrating the organic layer under reduced pressure, (S)-4-pentenoic acid derivative (148.4 g, 840 mmol) was obtained. The yield from the (S)-4-pentenoic acid derivative-(L)-lysine salt, was 99%.

Into a glass eggplant type flask, the (S)-4-pentenoic acid derivative (148.0 g, 838 mmol) was introduced, and methanol (110 mL) and 98% sulfuric acid (41.2 mL) were further added. With stirring, the mixture was heated at 85°C for 20 hours. Methanol was distilled off under reduced pressure, followed by extraction with toluene (225 mL). After washing the organic layer with water (150 mL × 5 times), it was concentrated under reduced pressure, and precision distillation was carried out under reduced pressure to obtain a methyl ester (122.9 g, 645 mmol) of (S)-4-pentenoic acid derivative. The yield from the (S)-4-pentenoic acid derivative-(L)-lysine salt, was 76%, and then it was analyzed by GC analysis, whereby the optical purity was 98.7% for S-isomer and 1.3% for R-isomer.

### EXAMPLE 10

Into a glass eggplant type flask, L-(+)-lysine monohydrate (2.7 g, 16.5 mmol) and 2.1 mL of water were introduced, and the racemic modification of 4-pentenoic acid derivative (5.3 g, 30 mmol) and 1,1-dimethyl ethanol (25 mL) were added. With stirring, the mixture was heated for refluxing at 90°C for 10 minutes to prepare a solvent solution. After completion of the heating, the solvent solution was left to cool to room temperature with stirring, whereby a white solid precipitated. The solvent solution was further stirred for 4 nights.

The white solid was collected by filtration and washed with 5% hydrous 1,1-dimethyl ethanol, and it was dried under reduced pressure to obtain (S)-4-pentenoic acid derivative-(L)-lysine salt (2.91 g, 9.01 mmol). The yield from the racemic modification was 30%.

The (S)-4-pentenoic acid derivative-(L)-lysine salt (20 mg) was subjected to the same preliminary treatment as in Example 4, and then it was analyzed by GC analysis, whereby the optical purity was 99.4% for S-isomer and 0.6% for R-isomer.

### EXAMPLE 11

Into a glass eggplant type flask, a 50% aqueous solution of L-(+)-lysine (4.8 g, 16.5 mmol) was introduced, and the racemic modification of 4-pentenoic acid derivative (5.3 g, 30 mmol) and acetone (40 mL) were added to prepare a solvent solution. With stirring, the solvent solution was heated for refluxing at 90°C for 30 minutes, whereby a white solid precipitated. After completion of the heating, a turbid solution of the white solid was left to cool to room temperature with stirring, and it was further stirred overnight at room temperature.

The white solid was collected by filtration and washed with 2% hydrous acetone, and it was dried under reduced pressure to obtain (S)-4-pentenoic acid derivative-(L)-lysine salt (2.95 g, 9.15 mmol). The yield from the racemic modification was 31%.

The obtained (S)-4-pentenoic acid derivative-(L)-lysine salt (20 mg) was subjected to the same preliminary treatment as in Example 4, and then it was analyzed by GC analysis, whereby the optical purity was 98.6% for S-isomer and 1.4% for R-isomer.

### EXAMPLE 12

Into a glass eggplant type flask, a 50% aqueous solution of L-(+)-lysine (3.95 g, 13.5 mmol) was introduced, and the racemic modification of 4-pentenoic acid derivative (5.3 g, 30 mmol) and acetone (30 mL) were added to prepare a solvent solution. When the solvent solution was stirred at room temperature, a white solid precipitated. The solvent solution was further stirred overnight.

The white solid was collected by filtration and washed with 2% hydrous acetone, and it was dried under reduced pressure to obtain (S)-4-pentenoic acid derivative-(L)-lysine salt (3.02 g, 9.36 mmol). The yield from the racemic modification was 31%.

The obtained (S)-4-pentenoic acid derivative-(L)-lysine salt (20 mg) was subjected to the same preliminary treatment as in Example 4, and then it was analyzed by GC analysis, whereby the optical purity was 99.2% for S-isomer and 0.8% for R-isomer.

### EXAMPLE 13

Into a glass eggplant type flask, a 50% aqueous solution of L-(+)-lysine (3.95 g, 13.5 mmol) was introduced, and the racemic modification of 4-pentenoic acid derivative (5.3 g, 30 mmol), 2-propanol (20 mL) and acetone (20 mL) were added to prepare a solvent solution. With stirring, the solvent solution was heated for refluxing at 80°C for 10 minutes, whereby a white solid precipitated. After completion of the heating, the white turbid solution was left to cool to room temperature with stirring. The solution was further stirred at around 5°C overnight.

The white solid was collected by filtration and washed with 2-propanol, and it was dried under reduced pressure to obtain (S)-4-pentenoic acid derivative-(L)-lysine salt (4.03 g, 12.5 mmol). The yield from the racemic modification was 42%.

The obtained (S)-4-pentenoic acid derivative-(L)-lysine salt (20 mg) was subjected to the same preliminary treatment as in Example 4, and then it was analyzed by GC analysis, whereby the optical purity was 98.7% for S-isomer and 1.3% for R-isomer.

### EXAMPLE 14

Into a glass eggplant type flask, a 50.8% aqueous solution of L-(+)-lysine (3.89 g, 13.5 mmol) was introduced, and the racemic modification of 4-pentenoic acid derivative (5.3 g, 30 mmol), 2-propanol (3.5 mL) and acetone (21 mL) were added to prepare a solvent solution. With stirring, the solvent solution was heated for refluxing at 70°C for 10 minutes, whereby a white solid precipitated. After completion of the heating, the white turbid solution was left to cool to room temperature with stirring. The solution was further stirred at around 5°C overnight.

The white solid was collected by filtration and washed with 2-propanol, and it was dried under reduced pressure to obtain (S)-4-pentenoic acid derivative-(L)-lysine salt (3.24 g, 10.0 mmol). The yield from the racemic modification was 33%.

The obtained (S)-4-pentenoic acid derivative-(L)-lysine salt (20 mg) was subjected to the same preliminary treatment as in Example 4, and then it was analyzed by GC analysis, whereby the optical purity was 99.2% for S-isomer and 0.8% for R-isomer.

### EXAMPLE 15

When the same treatment as in Example 14 was carried out by using the racemic modification of 4-pentenoic acid derivative (100.0 g, 566 mmol), (S)-4-pentenoic acid derivative-(L)-lysine salt (58.03 g, 180 mmol) was obtained. The yield from the racemic modification was 32%.

The obtained (S)-4-pentenoic acid derivative-(L)-lysine salt (20 mg) was subjected to the same preliminary treatment as in Example 4, and then it was analyzed by GC analysis, whereby the optical purity was 99.0% for S-isomer and 1.0% for R-isomer.

Into a glass eggplant type flask, the (S)-4-pentenoic acid derivative-(L)-lysine salt (57.53 g, 178 mmol) was introduced, and 15.5% sulfuric acid (153 mL) was further added, followed by extraction with toluene (133 mL × twice). The organic layer was concentrated under reduced pressure to obtain (S)-4-pentenoic acid derivative (31.2 g, 176 mmol). The yield from the racemic modification of 4-pentenoic acid derivative, was 31%.

Into a glass eggplant type flask, the (S)-4-pentenoic acid derivative (30.9 g, 175 mmol) was introduced, and methanol (21 mL) and 98% sulfuric acid (8.6 mL) were further added. With stirring, the mixture was heated at 85°C for 8 hours. Methanol was distilled off under reduced pressure, followed by extraction with toluene (47 mL). After washing the organic layer with a 6% of sodium hydrogencarbonate aqueous solution (32 mL) and water (32 mL), it was concentrated under reduced pressure, and precision distillation was further carried out under reduced pressure to obtain a methyl ester (27.7 g, 145 mmol) of (S)-4-pentenoic acid derivative. The yield from the racemic modification of 4-pentenoic acid derivative, was 26%. As a result of GC analysis, the optical purity was 99.1% for S-isomer and 0.9% for R-isomer.

### EXAMPLE 16

Into a glass eggplant type flask, L-(+)-lysine monohydrate (2.22 g, 13.5 mmol) and 1.07 mL of water were introduced (corresponding to a 60% aqueous solution of L-(+)-lysine), and the racemic modification of 4-pentenoic acid derivative (5.3 g, 30 mmol), 2-propanol (3.5 mL) and acetone (21 mL) were added, to prepare a solvent solution. With stirring, the solvent solution was heated for refluxing at 70°C for 15 minutes, whereby a white solid precipitated. After completion of the heating, the white turbid solution was left to cool to room temperature with stirring. The solution was further left to stand at around 5°C overnight.

The white solid was collected by filtration and washed with 2-propanol, and it was dried under reduced pressure to obtain (S)-4-pentenoic acid derivative-(L)-lysine salt (3.56 g, 11.0 mmol). The yield from the racemic modification was 37%.

The obtained (S)-4-pentenoic acid derivative-(L)-lysine salt (20 mg) was subjected to the same preliminary treatment as in Example 4, and then it was analyzed by GC analysis, whereby the optical purity was 98.8% for S-isomer and 1.2% for R-isomer.

### INDUSTRIAL APPLICABILITY

By the method of the present invention, it is possible to produce an optically active 4-pentenoic acid derivative, 4-pentenoic acid derivative salt or an optically active 4-pentenoic acid ester derivative with high yield and high optical purity. The obtained optically active 4-pentenoic acid derivative, 4-pentenoic acid derivative salt or optically active 4-pentenoic acid ester derivative, particularly S-isomer, is useful as an intermediate for e.g. agrochemicals or medicines.

Further, the method of the present invention does not require any special facility, device or operation, and it is capable of producing the above derivative by simple operation, and thus, it is useful as an industrial method.

## Claims

1. A method for producing a basic amino acid salt of optically active (4E)-5-chloro-2-isopropyl-4-pentenoic acid, which comprises precipitating the basic amino acid salt of optically active (4E)-5-chloro-2-isopropyl-4-pentenoic acid from a solvent solution containing an optical isomer mixture of (4E)-5-chloro-2-isopropyl-4-pentenoic acid and an optically active basic amino acid or a salt thereof.

2. The method according to Claim 1, wherein the solvent solution is a solvent solution formed by dissolving the optical isomer mixture of (4E)-5-chloro-2-isopropyl-4-pentenoic acid and the optically active basic amino acid or a salt thereof, in a solvent, or a solvent solution formed by dissolving, in the solvent, a product formed by reacting the optically active basic amino acid or a salt thereof with the optical isomer mixture of (4E)-5-chloro-2-isopropyl-4-pentenoic acid.

3. The method according to Claim 1 or 2, wherein the optical isomer mixture of (4E)-5-chloro-2-isopropyl-4-pentenoic acid is a racemic modification of (4E)-5-chloro-2-isopropyl-4-pentenoic acid.

4. The method according to any one of Claims 1 to 3, wherein the optically active basic amino acid is L-(+)-lysine.

5. The method according to any one of Claims 1 to 4, wherein the optically active (4E)-5-chloro-2-isopropyl-4-pentenoic acid is S-isomer.

6. The method according to any one of Claims 1 to 5, wherein the solvent solution is a solution of solvent containing an alcohol.

7. The method according to any one of Claims 1 to 5, wherein the solvent solution is a solution of solvent containing a ketone.

8. The method according to any one of Claims 1 to 5, wherein the solvent solution is a solution of solvent containing an alcohol and a ketone.

9. The method according to any one of Claims 1 to 8 further comprising the step of dissolving the basic amino acid salt of optically active (4E)-5-chloro-2-isopropyl-4-pentenoic acid in a solvent to obtain a solvent solution of the basic amino acid salt of optically active (4E)-5-chloro-2-isopropyl-4-pentenoic acid, and then, precipitating from the solvent solution, the basic amino acid salt of optically active (4E)-5-chloro-2-isopropyl-4-pentenoic acid.

10. The method according to any one of Claims 1 to 9 further comprising subjecting the basic amino acid of optically active (4E)-5-chloro-2-isopropyl-4-pentenoic acid to a desalting reaction to yield an optically active (4E)-5-chloro-2-isopropyl-4-pentenoic acid.

11. The method according to Claim 10 further comprising reacting an optically active basic amino acid with the optically active (4E)-5-chloro-2-isopropyl-4-pentenoic acid.

12. A method for producing an optically active (4E)-5-chloro-2-isopropyl-4-pentenoic acid, which comprises subjecting a salt of optically active (4E)-5-chloro-2-isopropyl-4-pentenoic acid with L-(+)-lysine, to a desalting reaction.

13. A method for producing (S)-(4E)-5-chloro-2-isopropyl-4-pentenoic acid, which comprises subjecting a salt of (S)-(4E)-5-chloro-2-isopropyl-4-pentenoic acid with L-(+)-lysine, to a desalting reaction.

14. The method according to any one of Claims 10 or 12 further comprising reacting the optically active (4E)-5-chloro-2-isopropyl-4-pentenoic acid with an alcohol to yield an optically active (4E)-5-chloro-2-isopropyl-4-pentenoic acid ester.

15. The method according to Claim 14, wherein the optically active (4E)-5-chloro-2-isopropyl-4-pentenoic acid is S-isomer.

16. A salt of optically active (4E)-5-chloro-2-isopropyl-4-pentenoic acid with a basic amino acid.

17. The salt according to Claim 16, wherein the salt is a salt of (S)-(4E)-5-chloro-2-isopropyl-4-pentenoic acid with L-(+)-lysine.

## Patentansprüche

1. Verfahren zur Herstellung eines basischen Aminosäuresalzes von optisch aktiver (4E)-5-Chlor-2-isopropyl-4-pentensäure, welches das Ausfällen des basischen Aminosäuresalzes von optisch aktiver (4E)-5-Chlor-2-isopropyl-4-pentensäure aus einer Lösungsmittellösung, enthaltend ein Gemisch optischer Isomere von (4E)-5-Chloro-2-isopropyl-4-pentensäure und eine optisch aktive basische Aminosäure oder ein Salz davon, umfaßt.

2. Verfahren gemäß Anspruch 1, wobei die Lösungsmittellösung eine Lösungsmittellösung ist, gebildet durch Lösen des Gemisches optischer Isomere von (4E)-5-Chlor-2-isopropyl-4-pentensäure und der optisch aktiven basischen Aminosäure oder eines Salzes davon in einem Lösungsmittel, oder eine Lösungsmittellösung, gebildet durch Lösen eines Produkts, gebildet durch Umsetzen der optisch aktiven basischen Aminosäure oder eines Salzes davon mit dem Gemisch optischer Isomere von (4E)-5-Chlor-2-isopropyl-4-pentensäure in dem Lösungsmittel, ist.

3. Verfahren gemäß Anspruch 1 oder 2, wobei das Gemisch optischer Isomere von (4E)-5-Chlor-2-isopropyl-4-pentensäure eine racemische Modifikation von (4E)-5-Chlor-2-isopropyl-4-pentensäure ist.

4. Verfahren gemäß einem der Ansprüche 1 bis 3, wobei die optisch aktive basische Aminosäure L-(+)-Lysin ist.

5. Verfahren gemäß einem der Ansprüche 1 bis 4, wobei die optisch aktive (4E)-5-Chlor-2-isopropyl-4-pentensäure S-Isomer ist.

6. Verfahren gemäß einem der Ansprüche 1 bis 5, wobei die Lösungsmittellösung eine Lösung von Lösungsmittel, enthaltend einen Alkohol, ist.

7. Verfahren gemäß einem der Ansprüche 1 bis 5, wobei die Lösungsmittellösung eine Lösung eines Lösungsmittels, enthaltend ein Keton, ist.

8. Verfahren gemäß einem der Ansprüche 1 bis 5, wobei die Lösungsmittellösung eine Lösung eines Lösungsmittels, enthaltend einen Alkohol und ein Keton, ist.

9. Verfahren gemäß einem der Ansprüche 1 bis 8, weiter umfassend den Schritt des Lösens des basischen Aminosäuresalzes optisch aktiver (4E)-5-Chlor-2-isopropyl-4-pentensäure in einem Lösungsmittel, um eine Lösungsmittellösung des basischen Aminosäuresalzes von optisch aktiver (4E)-5-Chlor-2-isopropyl-4-pentensäure zu erhalten, und anschließend das Ausfällen aus der Lösungsmittellösung des basischen Aminosäuresalzes von optisch aktiver (4E)-5-Chlor-2-isopropyl-4-pentensäure.

10. Verfahren gemäß einem der Ansprüche 1 bis 9, weiter umfassend das Unterwerfen der basischen Aminosäure von optisch aktiver (4E)-5-Chlor-2-isopropyl-4-pentensäure einer Entsalzungsreaktion, um optisch aktive (4E)-5-Chlor-2-isopropyl-4-pentensäure zu erhalten.

11. Verfahren gemäß Anspruch 10, weiter umfassend das Umsetzen einer optisch aktiven basischen Aminosäure mit der optisch aktiven (4E)-5-Chlor-2-isopropyl-4-pentensäure.

12. Verfahren zur Herstellung einer optisch aktiven (4E)-5-Chlor-2-isopropyl-4-pentensäure, welches das Unterwerfen eines Salzes optisch aktiver (4E)-5-Chlor-2-isopropyl-4-pentensäure mit L-(+)-Lysin einer Entsalzungsreaktion umfaßt.

13. Verfahren zur Herstellung von (S)-(4E)-5-Chlor-2-isopropyl-4-pentensäure, welches das Unterwerfen eines Salzes von (S)-(4E)-5-Chlor-2-isopropyl-4-pentensäure mit L-(+)-Lysin einer Entsalzungsreaktion umfaßt.

14. Verfahren gemäß einem der Ansprüche 10 oder 12, weiter umfassend das Umsetzen der optisch aktiven (4E)-5-Chlor-2-isopropyl-4-pentensäure mit einem Alkohol, um einen optisch aktiven (4E)-5-Chlor-2-isopropyl-4-pentensäureester zu erhalten.

15. Verfahren gemäß Anspruch 14, wobei die optisch aktive (4E)-5-Chlor-2-isopropyl-4-pentensäure S-Isomer ist.

16. Salz von optisch aktiver (4E)-5-Chlor-2-isopropyl-4-pentensäure mit einer basischen Aminosäure.

17. Salz gemäß Anspruch 16, wobei das Salz ein Salz von (S)-(4E)-5-Chlor-2-isopropyl-4-pentensäure mit L-(+)-Lysin ist.

## Revendications

1. Procédé pour produire un sel d'acide aminé basique d'acide (4E)-5-chloro-2-isopropyl-4-penténoïque optiquement actif, qui comprend la précipitation du sel d'acide aminé basique d'acide (4E)-5-chloro-2-isopropyl-4-penténoïque optiquement actif à partir d'une solution de solvant contenant un mélange d'isomères optiques d'acide (4E)-5-chloro-2-isopropyl-4-penténoïque et d'un acide aminé basique optiquement actif ou d'un sel de celui-ci.

2. Procédé selon la revendication 1, dans lequel la solution de solvant est une solution de solvant formée par dissolution du mélange d'isomères optiques d'acide (4E)-5-chloro-2-isopropyl-4-penténoïque et de l'acide aminé basique optiquement actif ou d'un sel de celui-ci, dans un solvant, ou une solution de solvant formée par dissolution, dans le solvant, d'un produit formé par réaction de l'acide aminé basique optiquement actif ou d'un sel de celui-ci avec le mélange d'isomères optiques d'acide (4E)-5-chloro-2-isopropyl-4-penténoïque.

3. Procédé selon la revendication 1 ou 2, dans lequel le mélange d'isomères optiques d'acide (4E)-5-chloro-2-isopropyl-4-penténoïque est une modification racémique d'acide (4E)-5-chloro-2-isopropyl-4-penténoïque.

4. Procédé selon l'une quelconque des revendications 1 à 3, dans lequel l'acide aminé basique optiquement actif est la L-(+)-lysine.

5. Procédé selon l'une quelconque des revendications 1 à 4, dans lequel l'acide (4E)-5-chloro-2-isopropyl-4-penténoïque optiquement actif est l'isomère S.

6. Procédé selon l'une quelconque des revendications 1 à 5, dans lequel la solution de solvant est une solution de solvant contenant un alcool.

7. Procédé selon l'une quelconque des revendications 1 à 5, dans lequel la solution de solvant est une solution de solvant contenant une cétone.

8. Procédé selon l'une quelconque des revendications 1 à 5, dans lequel la solution de solvant est une solution de solvant contenant un alcool et une cétone.

9. Procédé selon l'une quelconque des revendications 1 à 8, comprenant en outre l'étape de dissolution du sel d'acide aminé basique de l'acide (4E)-5-chloro-2-isopropyl-4-penténoïque optiquement actif dans un solvant pour obtenir une solution de solvant du sel d'acide aminé basique d'acide (4E)-5-chloro-2-isopropyl-4-penténoïque optiquement actif, et ensuite l'étape de précipitation, à partir de la solution de solvant, le sel d'acide aminé basique d'acide (4E)-5-chloro-2-isopropyl-4-penténoïque optiquement actif.

10. Procédé selon l'une quelconque des revendications 1 à 9, comprenant en outre la soumission de l'acide aminé basique d'acide (4E)-5-chloro-2-isopropyl-4-penténoïque optiquement actif à une réaction de dessalage pour engendrer un acide (4E)-5-chloro-2-isopropyl-4-penténoïque optiquement actif.

11. Procédé selon la revendication 10, comprenant en outre la réaction d'un acide aminé basique optiquement actif avec l'acide (4E)-5-chloro-2-isopropyl-4-penténoïque optiquement actif.

12. Procédé pour produire un acide (4E)-5-chloro-2-isopropyl-4-penténoïque optiquement actif, qui comprend la soumission d'un sel d'acide (4E)-5-chloro-2-isopropyl-4-penténoïque optiquement actif et de L-(+)-lysine à une réaction de dessalage.

13. Procédé pour produire de l'acide (S)-(4E)-5-chloro-2-isopropyl-4-penténoïque, qui comprend la soumission d'un sel d'acide (S)-(4E)-5-chloro-2-isopropyl-4-penténoïque et de L-(+)-lysine à une réaction de dessalage.

14. Procédé selon l'une quelconque des revendications 10 à 12, comprenant en outre la réaction de l'acide (4E)-5-chloro-2-isopropyl-4-penténoïque optiquement actif avec un alcool pour engendrer un ester d'acide (4E)-5-chloro-2-isopropyl-4-penténoïque optiquement actif.

15. Procédé selon la revendication 14, dans lequel l'acide (4E)-5-chloro-2-isopropyl-4-penténoïque optiquement actif est l'isomère S.

16. Sel d'acide (4E)-5-chloro-2-isopropyl-4-penténoïque optiquement actif et d'un acide aminé basique.

17. Sel selon la revendication 16, lequel sel est un sel d'acide (S)-(4E)-5-chloro-2-isopropyl-4-penténoïque et de L-(+)-lysine.
